# EUROPEAN PATENT APPLICATION

(11) **EP 1 210 935 A1**
(43) Date of publication of application: **05.06.2002**
(21) Application number: 00954962.7
(22) Date of filing: 24.08.2000
(51) Int. Cl.: A61K 9/12, A61K 47/34, A61K 47/46, A61K 47/26, A61K 47/14, A61K 47/44, A61K 47/20

(54) **MEDICINAL COMPOSITIONS FOR ORAL USE**

(30) Priority: 03.09.1999 JP 25083799
(71) Applicant: Kissei Pharmaceutical Co., Ltd., Matsumoto-shi Nagano 399-8710 (JP)
(72) Inventor: OKUBO, Yoshio, Nerima-ku, Tokyo 176-0023 (JP); MORIYA, Hidetaka, Matsumoto-shi, Nagano 390-0851 (JP); SAITO, Noriyasu, Shiojiri-shi, Nagano 399-0706 (JP); YUASA, Hiroshi, Shibuya-ku, Toyko 151-0072 (JP)
(74) Representative: Baverstock, Michael George Douglas
(86) International application number: JP0005677
(87) International publication number: WO0117505

(57) **Abstract**

The present invention relates to an oral medicinal composition comprising an active ingredient and at least one foaming agent, which is ejected from a foam-developing device to be prepared into foam, and a method for administering an oral medicinal composition comprising an active ingredient and at least one foaming agent, which is ejected from a foam-developing device to prepare the oral medicinal composition into foam for administration, the composition being preferably applicable to patients with deglutition disorder or persons with swallowing difficulty.

## Description

### Technical Field

The present invention relates to an oral medicinal composition suitable for administration to patients having difficulty in swallowing, such as patients with deglutition disorder, aged persons, children or patients needing rest post-surgery, and a method for administering the oral medicinal composition.

### Background of the Invention

Previously, solid dosage forms such as capsules, tablets, granules and powders as well as liquid dosage forms such as liquids for internal use, suspensions, emulsions, syrups, elixirs, aromatic waters and lemonades, have been generally used as oral pharmaceutical dosage forms. However, no foamed dosage forms have been used orally yet.

As foamed dosage forms, alternatively, foamed aerosols have been commonly used for cosmetic products and external preparations. However, such foamed dosage forms have never been used as oral pharmaceutical dosage forms. Additionally, for the route of administration into the oral cavity or throat, powders, liquids for internal use, suspensions and the like have been partly administered by modifying them into aerosols or into the form of a fog or drops by using an appropriate device. Nevertheless, no method for modifying them into foam for oral administration has been used yet.

When patients with difficulty in swallowing or not good at swallowing, such as patients with deglutition disorder, aged persons, children or patients needing rest post-surgery, intend to take oral pharmaceutical dosage forms of the related art, the patients have encountered various problems during administration, because solid dosage forms such as general capsules, tablets, granules and powders per se involve difficulty in swallowing and therefore require water during dosing and because these dosage forms should simultaneously be poured along with water into the throat, which sometimes causes an episode of choking.

When patients are lying on their sides in bed, such as patients needing rest post-surgery and the like and bedridden patients, to take such solid dosage forms, it is difficult for the patients to swallow the dosage forms *per se.* Further, these solid dosage forms readily enter the airway, which induces additional pain.

Additionally, patients at the last stage of cancer and patients with severe oral herpes simplex complain of unendurable pain in contact with liquids, to say nothing of solids. Therefore, serious disadvantages have been induced in their daily lives.

So far, dosage forms of orally disintegrating type have been developed, which can be administered with a small volume of water and are suitable for persons with swallowing difficulty including patients with deglutition disorder or aged persons. However, all of the dosage forms are solid dosage forms, so the dosage forms differ significantly from the foamed dosage form of the invention.

Meanwhile, even relatively readily incorporable liquid dosage forms such as liquids for internal use, suspensions, emulsions, syrups, elixirs, aromatic waters and lemonades may happen to be rapidly poured into the throat, so the liquid dosage forms also readily enter the airway, like solid dosage forms to be poured along with water. Thus, such liquid dosage forms may occasionally be never assimilated, because of an episode of choking.

As described above, additionally, even liquid contact causes strong physical irritation, so such liquid dosage forms are not sufficiently satisfactory for patients at the last stage of cancer or patients with severe oral herpes simplex, with complaints of unendurable pain.

In the case of liquid dosage forms, furthermore liquid dosage forms should be measured, at the cost of complicated labor, before administration. Additionally, the measurement of a dose in a constant manner has not been so simple; a disadvantage.

When the liquid dosage forms of the related art need to be applied at a topical site, such as the throat, furthermore, the liquid dosage forms can rapidly reach the site where the pharmaceutical component therein can be absorbed or a site to be therapeutically treated, but the liquid dosage forms also rapidly pass through such sites, disadvantageously, with no possibility of sufficient therapeutic effect.

### Disclosure of the Invention

The invention relates to an oral medicinal composition comprising an active ingredient and at least one foaming agent, which is ejected from a foam-developing device to be prepared into foam.

Additionally, the invention relates to a method for administering an oral medicinal composition comprising an active ingredient and at least one foaming agent, which is ejected from a foam-developing device to prepare them into foam for administration.

### Best Mode for Carrying out the Invention

A problem of the invention is to develop a dosage form administrable to patients at the last stage of cancer and patients with severe oral herpes simplex, which can be dosed with no need of water and can be readily swallowed but can never be rapidly poured into the throat, involving less chance of entering the airway and no occurrence of choking, and which causes less physical irritation, and a method for administering such dosage form.

Further, a problem of the invention is to develop a dosage form which can be dosed routinely at a constant amount with no need of complicated measurement and which can be readily incorporated by persons lying on their sides in bed and can be retained sufficiently at a site in the oral cavity or throat where the pharmaceutical component therein can be absorbed or a site to be therapeutically treated, and a method for administering such dosage form.

The present inventors have made intensive investigations so as to develop an oral medicinal composition which can overcome the problem and which is suitable for dosing to patients with deglutition disorder or persons with swallowing difficulty, and a method for administering such oral medicinal composition. Consequently, the inventors have found that by preparing oral liquid dosage forms such as liquids for internal use, suspensions, emulsions, syrups, elixirs, aromatic waters and lemonades into foam for administration, to allow the resulting foam to again resume the liquid forms after administration, the oral liquid dosage forms can be dosed with no water, be readily swallowed with no rapid pouring into the throat and no subsequent passage into the airway causing an episode of choking, has less physical irritation and can be dosed readily in a safe manner. Thus, the invention has been achieved.

More specifically, the inventors have found that by modifying an oral medicinal composition comprising an active ingredient and at least one foaming agent, which is turned into foam when ejected from a foam-developing device, into foam via release of the oral medicinal composition from an appropriate foam-developing device and then administering the resulting foam from the foam-developing device, the oral medicinal composition never causes any irritation at the time of dosing, or never requires the supply of extra water or specific effort for swallowing. Further, the oral medicinal composition can be spontaneously poured into the throat without rapid influx into the throat or without influx into the airway which induces choking and is extremely suitable for administration to patients with deglutition disorder or persons with swallowing difficulty.

By measuring a given amount of the medicinal composition into a foam-developing device to allow the medicinal composition to be prepared into foam for administration, additionally, no complicated measurement is required before dosing and a constant dose can be given routinely.

Characteristically, the oral medicinal composition of the invention comprises an active ingredient and at least one foaming agent and is prepared into foam when ejected from a foam-developing device. By foaming the oral medicinal composition prior to administration and then gradually allowing the foamed composition to resume the initial liquid form, the oral medicinal composition can be extremely readily administered with no irritation during administration, without any water supply, with no chance of a choking episode due to the introduction of the oral medicinal composition or water into the airway during dosing, and with no complicated measurement prior to dosing, and the oral medicinal composition can be routinely dosed readily at a constant dose. Thus, the oral medicinal composition exerts innovative effects.

Any foaming agent may be used for the oral medicinal composition of the invention, provided that the foaming agent can prepare non-irritable foam, without interactions such as incompatible combination with the active ingredient and pharmaceutical additives to be contained therein, being capable of long-term storage and being appropriately self-sustainable when ejected from a foam-developing device. The foaming agents include, for example, polyethylene glycol, stearyl alcohol, saponin, propylene glycol, medium-chain fatty acid triglyceride, cacao butter, sucrose esters of fatty acids, polyoxyl stearate, polyoxyethylene hydrogenated castor oil, polyoxyethylene polyoxypropylene glycol, sorbitan sesquioleate, sorbitan trioleate, sorbitan monolaurate, polysorbate, glyceryl monostearate, sodium lauryl sulfate, lauromacrogol and the like.

Among these foaming agents, polysorbate, polyethylene glycol, and sodium lauryl sulfate are preferably illustrated. A mixture of polyethylene glycol with polysorbate or sodium lauryl sulfate is particularly preferable.

These foaming agents may be added singly or in a combination of such plural foaming agents. The blend quantity is appropriately determined depending on the physico-chemical properties of the active ingredient and pharmaceutical additives contained therein and the properties of the foaming agent. Approximately, the foaming agent may be satisfactorily blended within a range of 1 to 20 % by weight to the whole oral medicinal composition.

Further, the properties of the resulting foam vary depending on the type of a foaming agent to be added. For example, when polyethylene glycol is used, the duration of the resulting self-sustainable foam is short. Also, when polysorbate and sodium lauryl sulfate are used, the duration of the resulting self-sustainable foam is long. The resulting self-sustainable foam is influenced by the mean molecular weight of the foaming agent. By preparing a combination of plural foaming agents by utilizing such properties and further modifying the blend ratio or blend quantities, the duration of the resulting self-sustainable foam can be adjusted depending on the applicable drug or the subject patient.

When the oral medicinal composition of the invention is to be applied as a routine pharmaceutical agent for internal use, self-sustainable foam of a relatively shorter duration can be liquefied more rapidly to be then poured down the throat. Therefore, such foam is preferable because the time required for incorporation is shorter. Preferably, the duration of self-sustainable foam is specifically about several seconds to several tens of seconds.

When the oral medicinal composition is to be applied into the oral cavity or throat, self-sustainable foam of a longer duration or a liquid with a higher viscosity has a longer retention time at a site where the pharmaceutical component therein can be absorbed or a site to be therapeutically treated. Thus, such foam or such liquid is preferable because sufficient therapeutic effects can be exerted. In particular, when the oral medicinal composition is to be applied into the oral cavity, the composition is preferably retained in the oral cavity in a still foaming state. Also, when the oral medicinal composition is to be applied into the throat, the composition is preferably poured into the throat while the composition still retains the foamy state.

As described above, the duration of self-sustainable foam can be adjusted by selecting a foaming agent or preparing a combination of such foaming agents. Preparation of an oral medicinal composition with a longer duration of self-sustainable foam, can be achieved by adding an appropriate viscous agent other than a foaming agent to permit the duration of self-sustainable foam to be longer.

Such viscous agents include, for example, acrylic resin alkanol amine solution, sodium alginate, propylene glycol alginate, carmellose sodium, xanthan gum, guar gum, glycerin, sodium chondroitin sulfate, purified lanolin, gelatin, dextrin, potato starch, hydroxyethylcellulose, hydroxyethylmethyl-cellulose, hydroxypropylcellulose, hydroxypropylmethyl-cellulose, polyvinylpyrrolidone, strach syrup, yellow beeswax, methylcellulose and the like.

The viscous agent can prolong the duration of self-sustainable foam and raise the viscosity of the resulting liquid, simultaneously, leading to the prolongation of the retention time after liquefaction. Thus, such viscous agent can be also used for the purpose of the local prolongation of the retention time after liquefaction.

These viscous agents can be added singly or in combination of such plural viscous agents. Additionally, the blend quantity is appropriately determined depending on the physico-chemical properties of the active ingredient and pharmaceutical additives to be contained in the initial oral pharmaceutical liquid dosage form and the properties of the viscous agents. Approximately, the blend quantity may be satisfactorily within a range of 1 to 10 % by weight to the whole oral medicinal composition.

The duration of self-sustainable foam varies depending on not only the kind, blend ratio and molecular weight of a foaming agent but also the kinds and amounts of various pharmaceutical additives such as sweeteners, aromatics, pH adjusting agents, buffering agents and stabilizers, which are appropriately added in a dependent manner on the kind of the solution and in terms of pharmaceutical preparation. Thus, the duration of self-sustainable foam should be essentially adjusted depending on the final formulation.

For preparing the oral medicinal composition of the invention, a foaming agent and, if necessary, a viscous agent are added together with the active ingredient and pharmaceutical additives according to general preparation methods. Otherwise, a routine oral pharmaceutical liquid dosage form is firstly prepared, to which a foaming agent and, if necessary, a viscous agent are added at the time of administration. When the active ingredient to be used is unstable when prepared in a liquid dosage form, satisfactorily, the liquid base without containing the active ingredient for the oral medicinal composition may be preliminarily prepared, to which the active ingredient is then added at the time of administration. Otherwise, the oral medicinal composition may be satisfactorily prepared into powders, such as freeze-dry powders, to which water is added at the time of administration to prepare the oral medicinal composition in liquid form.

If necessary, additionally, corrigents or aromatics for use in general formulations may be satisfactorily added. Still further, pH adjusting agents, buffering agents, stabilizers and the like to keep the solubility and stability may be satisfactorily added depending on the characteristic of the active ingredient.

So as to administer practically the oral medicinal composition of the invention, an oral medicinal composition comprising an active ingredient and at least one foaming agent, which is prepared by a general method and can be turned into foam when ejected from a foam-developing device, is dosed by means of an appropriate foam-developing device; otherwise, a foaming agent and, if necessary, a viscous agent are added to a general oral pharmaceutical liquid dosage form preliminarily prepared, which is then dosed by means of the foam-developing device. When the active ingredient is unstable when prepared in a liquid dosage form, a liquid base without containing the active ingredient for an oral medicinal composition is preliminarily prepared, to which the active ingredient is then added at the time of administration. The resulting formulation may be satisfactorily dosed by means of the foam-developing device.

Through the administration by means of a foam-developing device as described above, any complicated procedure for measuring the dose as in the case of dosing general oral pharmaceutical liquid dosage forms can be saved; additionally, a constant volume can be taken consistently; and dosing can be done in a very easy and safe manner.

The foam-developing device for use in the administration of the oral medicinal composition of the invention may be any foam-developing device with a function to permit the preparation of a given amount of the oral medicinal composition into foam, with no specific limitation. Preferable such foam-developing devices include an aerosol container with a quantitative dosing valve or a container for mixing a given amount of air with a given amount of composition and releasing then the resulting mixture through porous materials such as mesh.

The oral medicinal composition of the invention is applicable to any active ingredient, which can be dissolved, emulsified or suspended in water and whose viscosity can be adjusted as required.

The oral medicinal composition is preferably applicable to pharmaceutical agents for subject patients with swallowing difficulty or with pain during swallowing, such as patients with deglutition disorder, aged persons, children or patients needing rest post-surgery, particularly, pharmaceutical agents such as topical anesthesia for subject patients at the last stage of cancer or patients with severe oral herpes simplex, or pharmaceutical agents to be essentially dosed without water, dosage forms for patients under water intake control, pharmaceutical agents for one draft, and the like. Further, pharmaceutical agents with relatively low doses are preferable. When a large dose is required, the dose can be administered in a divided manner.

### Examples

The features of the invention will be illustrated in more detail by way of the following Examples and Reference Examples. However, the invention is not limited to their contents.

### Example 1

Using sodium lauryl sulfate and polyethylene glycol (Macrogol 6000) as foaming agents, liquids with variable blend ratios were prepared. Distilled water was used as a control. Under observation of foaming, the duration of the resulting self-sustainable foam vs. the change of the blend ratio was measured. For test solutions, sodium lauryl sulfate was blended in the individual ratios in Table 1 below, provided that the concentration of polyethylene glycol was fixed to 10 mg/mL, which was defined as 100. The duration of self-sustainable foam was measured according to the following method (hereinafter referred to as Method A). After ejecting a liquid from a foam-developing device to allow foam to be ejected onto a 30cm height position of a plastic plate in vertical position, the time (second) required for the foam to be subsequently liquefied and flow down until the top of the liquid reached the lower end of the plate was measured, which was designated the duration of self-sustainable foam. The test was repeated three times. The mean was determined. The results measured are shown below in Table 1. Distilled water (control) never made any foam. The duration of self-sustainable foam was prolonged by increasing the amount of sodium lauryl sulfate.

**[Table 1]**

| | Control (distilled water) | Formulation 1 | Formulation 2 | Formulation 3 |
|---|---|---|---|---|
| Sodium lauryl sulfate | 0 | 0 | 0.05 | 0.5 |
| Macrogol 6000 | 0 | 100 | 100 | 100 |
| Duration of self-sustainable foam (second) | - | 9 | 53 | 84 |

### Example 2

Using polysorbate 80 and polyethylene glycol (Macrogol 6000) as foaming agents, liquids with variable blend ratios were prepared. As in Example 1, the duration of the resulting self-sustainable foam vs. the change of the blend ratio was measured under observation of foaming. For test solutions, polysorbate 80 was blended to in individual ratios in Table 2 below, provided that the concentration of polyethylene glycol was fixed to 10 mg/mL, which was defined as 100. The duration of self-sustainable foam was measured by the Method A as in Example 1. The test was repeated three times. The mean was determined. The results measured are shown below in Table 2. The duration of self-sustainable foam was prolonged by increasing the amount of polysorbate 80.

**[Table 2]**

| | Formulation 1 | Formulation 4 | Formulation 5 |
|---|---|---|---|
| Polysorbate 80 | 0 | 5 | 20 |
| Macrogol 6000 | 100 | 100 | 100 |
| Duration of self-sustainable foam (second) | 9 | 31 | 41 |

### Example 3

Using sodium lauryl sulfate and polyethylene glycol (Macrogol 6000) as foaming agents as in Example 1, liquids with variable blend ratios between sodium lauryl sulfate and Macrogol 6000 were prepared. The duration of the resulting self-sustainable foam vs. the change of the blend ratio was measured under observation of foaming. For test solutions, the total of sodium lauryl sulfate and Macrogol 6000 was adjusted to 0.5 w/w %. For the purpose of measuring the flow through the oral cavity and throat, the duration of self-sustainable foam was measured according to the following method (hereinafter referred to as Method B). After measuring 10 mL of a liquid and ejecting the total volume from a foam-developing device to make the foam on a funnel connected to a measuring cylinder. the time (seconds) required for the foam to flow down the funnel to move into the measuring cylinder until 50 % of the foam was liquefied (a liquid volume of 5 mL) was measured, which was designated the duration of self-sustainable foam. The test was repeated three times. The mean was determined. The results measured are shown below in Table 3. In the case of sodium lauryl sulfate alone (Formulation 9), the duration of self-sustainable foam was prolonged and was about 10-fold that of the duration in case of Macrogol 6000 alone (Formulation 6), and the duration of self-sustainable foam was prolonged by increasing the amount of sodium lauryl sulfate.

**[Table 3]**

| | Formulation 6 | Formulation 7 | Formulation 8 | Formulation 9 |
|---|---|---|---|---|
| Sodium lauryl sulfate | 0 | 10 | 50 | 100 |
| Macrogol 6000 | 100 | 90 | 50 | 0 |
| Duration of self-sustainable foam (second) | 23 | 143 | 199 | 256 |

### Example 4

Using sodium lauryl sulfate and polyethylene glycol (Macrogol 6000) as foaming agents as in Example 1, liquids were prepared so that the total of the two foaming agents might be 10 mg/mL. For preparing liquids, distilled water was used for one formulation (Formulation 10), while McIlvaine's buffer solution, pH 7 was used for the other formulation (Formulation 11). The duration of self-sustainable foam was measured by exactly the same method as in Example 1. As the results are shown below in Table 4, the duration of self-sustainable foam was shortened when using the buffer solution.

**[Table 4]**

| | Formulation 10 | Formulation 11 |
|---|---|---|
| Preparative solution | distilled water | McIlvaine's buffer solution, pH 7 |
| Sodium lauryl sulfate | 0.5 | 0.5 |
| Macrogol 6000 | 99.5 | 99.5 |
| Duration of self-sustainable foam (second) | 85 | 13 |

### Reference Example 1

### Stability test

The liquid of the Formulation 11 as prepared in Example 4 was stored in a glass container at 5 °C, 40 °C and 60 °C for 2 weeks. Then, the appearance of the liquid was observed, while the change of the duration of self-sustainable foam was measured. The duration of self-sustainable foam was measured by the Method A of Example 1 and the Method B of Example 3. As shown in the results below in Table 5, the liquid was stable with no significant change of the appearance or the duration of self-sustainable foam.

**[Table 5]**

| Storage conditions | Time of preparation | 5 °C | 40 °C | 60 °C |
|---|---|---|---|---|
| Appearance | colorless, transparent | colorless, transparent | colorless, transparent | colorless, transparent |
| Duration of self-sustainable foam as measured by Method A (seconds) | 13 | 14 | 15 | 13 |
| Duration of self-sustainable foam as measured by Method B (in seconds) | 56 | 56 | 66 | 64 |

### Reference Example 2

### Dose uniformity test

Adding an active ingredient to the liquid of the Formulation 11 of Example 4 for adjustment to the concentration of 1.25 mg/mL and continuously ejecting the resulting mixture from a foam-developing device (at an ejection volume of 0.8 mL/one time) 10 times for foaming, the content of the active ingredient in the individuals was measured. As the results are shown below in Table 6, the content of active ingredient was almost constant.

**[Table 6]**

| n | 1 | 2 | 3 | 4 | 5 | 6 | 7 | 8 | 9 | 10 |
|---|---|---|---|---|---|---|---|---|---|---|
| Content (mg) | 0.93 | 0.95 | 0.92 | 0.94 | 0.94 | 1.02 | 0.98 | 0.99 | 0.97 | 0.97 |

Mean content: 0.96 mg
RSD: 3.2 %

### Industrial Applicability

The medicinal composition of the invention can be dosed even to patients at the last stage of cancer and patients with severe oral herpes simplex, because the composition can be dosed without water and easily swallowed with no rapid flow into the throat involving less concern of passage into airway and no occurrence of choking and because the composition induces less physical irritation. Additionally, the medicinal composition of the invention can be dosed at a constant amount, involving no complicated measuring procedure, can be readily dosed to persons lying on their sides in bed, and can be retained sufficiently at a site in the oral cavity or throat where the pharmaceutical component therein can be absorbed or at a site to be therapeutically treated. Thus, the medicinal composition of the invention is preferable for administration to patients with deglutition disorder or persons with swallowing difficulty.

## Claims

1. An oral medicinal composition comprising an active ingredient and at least one foaming agent, which is ejected from a foam-developing device to be prepared into foam.

2. An oral medicinal composition according to claim 1, where the foaming agent is at least one selected from the group consisting of polyethylene glycol, saponin, sucrose esters of fatty acids, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate and lauromacrogol.

3. An oral medicinal composition according to claim 2, where the foaming agent is at least one selected from the group consisting of polysorbate, polyethylene glycol and sodium lauryl sulfate.

4. An oral medicinal composition according to claim 3, where the foaming agent is a mixture of polyethylene glycol and polysorbate or a mixture of polyethylene glycol and sodium lauryl sulfate.

5. A method for administering an oral medicinal composition, which comprises ejecting an oral medicinal composition comprising an active ingredient and at least one foaming agent from a foam-developing device to prepare the oral medicinal composition into foam for administration.

6. A method for administering an oral medicinal composition according to claim 5, where the foaming agent is at least one selected from the group consisting of polyethylene glycol, saponin, sucrose esters of fatty acids, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate and lauromacrogol.

7. A method for administering an oral medicinal composition according to claim 6, where the foaming agent is at least one selected from the group consisting of polysorbate, polyethylene glycol and sodium lauryl sulfate.

8. A method for administering an oral medicinal composition according to claim 7, where the foaming agent is a mixture of polyethylene glycol and polysorbate or a mixture of polyethylene glycol and sodium lauryl sulfate.
